# EUROPEAN PATENT SPECIFICATION

(11) **EP 2 488 239 B1**
(45) Date of publication and mention of the grant of the patent: **09.09.2015**
(21) Application number: 10776439.1
(22) Date of filing: 22.09.2010
(51) Int. Cl.: A61M 11/02, A61M 15/00, A61M 15/08, A61M 11/06

(54) **NEBULIZER FOR AEROSOLTHERAPY**
ZERSTÄUBER FÜR AEROSOLTHERAPIE
ATOMISEUR POUR AÉROSOLTHÉRAPIE

(30) Priority: 15.10.2009 IT BS20090030 U
(43) Date of publication of application: 22.08.2012
(73) Proprietor: Flaem Nuova S.p.A., 25010 S. Martino Della Battaglia (BS) (IT)
(72) Inventor: ABATE, Luigi, I-25010 S. Martino Della Battaglia (BS) (IT); ABATE, Ricardo, I-25010 S. Martino Della Battaglia (IT)
(74) Representative: Sangiacomo, Ines
(86) International application number: PCT/IT2010/000400
(87) International publication number: WO 2011/045827

(56) References cited:
- EP-A1- 0 267 428
- EP-A1- 1 859 828
- EP-A2- 0 261 649
- WO-A1-2008/053666
- US-A- 3 580 249

## Description

This invention concerns the sector for apparatus for the nebulisation of a remedy for aerosol therapy, and refers in particular to an ampoule nebuliser to be used with said apparatus and provided with a nosepiece, a mask or a nasal adapter for the inhalation of a nebulised medicament on the part of the patient

Various embodiments and configurations of ampoules for nebulisation systems of a medicinal liquid to be inhaled to cure pathologies or respiratory system illnesses are already well known.

The type of ampoule nebuliser taken into consideration usually comprises, also corresponding to the preamble of claim 1:
a body that can be made up of two or more complementary elements and that define a chamber designed to contain a medicinal liquid to be nebulised,
a conduit extending upwards from the bottom of said chamber, designed for the input of a flow of compressed air, terminating with a top orifice and in addition a nebuliser nozzle possibly overlooked by a jet atomizer cylinder, generally called an atomizer or "pisper",
a through conduct, open both towards the external and towards the nebuliser nozzle for the feed of an additional flow of ambient air needed to increase the delivery speed of the remedy, and
a delivery conduit that extends from said chamber towards the outside to deliver the atomised medicament towards a patient.

Compared to the longitudinal axis of the ampoule, the output conduit of the medicament can be vertical or sloping and protruding from a side of the body of the ampoule, and to it may be attached a nosepiece, a mask or a nasal adaptor to improve the inhalation of the medicament by the patient.

The mixing and the atomizing of the liquid medicament with the air takes place by means of the nebuliser nozzle. Here, a flow of air under pressure, coming from a compressor, which arrives from the bottom to the conduit directed upwards in the chamber, contributes by the Venturi effect to the aspiration of the medicament from said chamber and to its atomization on a level with the "pisper".

It is well known that in said ampoule nebulisers, by varying the flow of ambient air that enters from outside through the delivery conduit, it is possible to adjust the delivery speed (if not also the size of the particles) of the medicament, the delivery speed being higher when, under the same conditions of the air under pressure supplied by the compressor, a greater flow of ambient air is added on entering.

However, according to the state of the technique some ampoule nebulisers are without any means able to vary the input of ambient air and t the atomization speed of the medicament. At the most, it is however possible to vary the input flow of ambient air by placing a finger on the entrance of the feed conduct with the drawback however of an adjustment that is highly aleatory.

Some other ampoule nebulizers have been provided, on a level with the ambient air feed cannel, with a throttle type valve that, however, can be opened in different but not definable sizes, with the disadvantage that the adjustment of the atomization speed becomes fortuitous.

Other ampoule nebulisers have been provided with a valve, or better with a cap, which is applied to the entrance of the feed channel when it is required to be blocked so as to lessen the amount of ambient air and to maintain the atomization speed at a minimum and which is completely removable so as to fully open the feed channel and maximize the automation speed of the medicament. Such a valve is not however easy to use and even if and when, as has taken place, it is connected to the body of the ampoule, it cannot be excluded that it may come loose and get lost.

The documents US 5875774, EP 0620021, EP 0627266, EP 1234591, and WO 2003/086081 are indicative of the state of the technique.

The principal object of this invention is to avoid the inconveniences and drawbacks of the known techniques and therefore to propose an ampoule nebuliser having at least two well defined supply speeds of the medicament controlled by a means easy to adjust, able to undertake at least two or more distinct positions each corresponding to a supply speed and which however remains constrained to the body of the ampoule.

Another object of the invention is provide an ampoule of the type and for the above mentioned use and equipped to allow an adjustment of the flow of medicament even at a very low working speed and to advantageously limit dispersion of medicine.

A further object of the invention is to propose an ampoule nebuliser for more efficient devices for aerosol therapy able to use the remedy medicament better for greater efficiency in the treatment of a patient.

According to a first aspect of the invention, such objects are achieved with an ampoule nebuliser according to the preamble of claim 1 and provided furthermore, on a level with the entry of the ambient air feed conduct, with a selection push-button moveable at least between a first extreme obstruction position of the entry of said conduct, for the choice of a first mobilization speed and a second open position of said conduit for the choice of a second delivery speed of the medicament.

Preferably and advantageously the selection push-button can also be provided for undertaking possible intermediate positions between said two extreme positions, for a partial variable opening of said conduct and a better adjustment of the delivery speed of the medicament.

In other words, the ampoule nebulizer becomes provided with a flow regulator/variator with which it will be possible to adjust the delivery speed of the medicament without substantial variations of the dimensions of the transported medicament particles or also to vary the dimensions of the medicament particles and the quantity of the fraction breathable.

According to another aspect of the invention an additional valve is associated with the selection push-button in order to increase the delivery of medicament when the atomization speed is minimum, thus ensuring the efficiency of the treatment being carried out also in this condition.

According to yet another aspect of the invention, the ampoule, provided with a flow regulator without or with an additional valve, can also be provided with an inhalation mask or mouthpiece having at least a dispersion limiter with variable width, which without influencing the inhalation and expiration of the patient makes possible however and advantageously the reduction of the dispersion of medicine in the intermediate periods between inhalation and expiration phases.

Further details will become evident from the description that follows made in reference to the attached indicative and not limiting drawings, in which:
Fig. 1 shows an exploded view of the components of an ampoule complete with a speed selector;
Fig. 2 shows a view in perspective of the assembled ampoule;
Fig. 3 shows a view from a side thereof;
Fig. 4 shows an axial cross section of the ampoule with the selection push-button in a first position;
Fig. 5 shows a similar axial cross section, but with the selection push-button in a second position; and
Fig. 6 shows an axial cross section according to the arrows A-A in Fig. 5;
Fig. 7 shows a cross section view of an ampoule having an additional valve associated with the speed variator and also complete with a inhalation mask;
Figs. 8 and 9 show an enlargement of the circled part in Fig. 7, with the additional valve in the closed and open positions, respectively;
Fig. 10 shows an isometric view of the ampoule with inhalation mask as in Fig. 7; and
Figs. 11 and 12 show the inhalation mask with dispersion limiter of the medicament in two different open positions, respectively in the inhalation and expiration phases of the patient.

The ampoule nebuliser has a body 10 made up of a lower element 11 and of a top cover 12, fixed between them in a separable form for example by means of a connection 13 by screwing or by bayonet, as shown in the drawings.

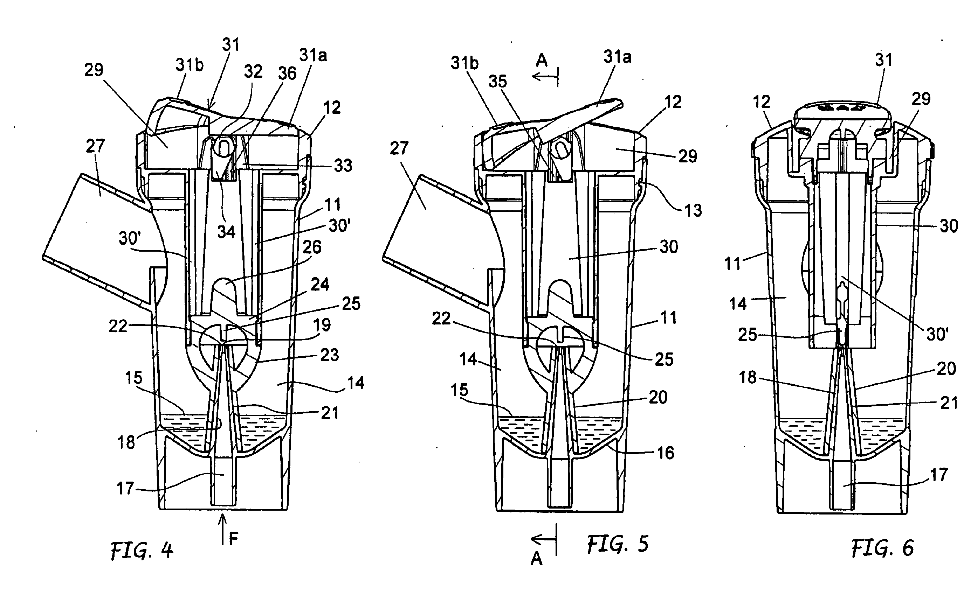

The lower element 11 delimits a chamber 14 for a liquid medicament 15 to atomize. Said chamber has a sloping bottom 16 crossed in the centre by a conduit 17 for air to enter under pressure, according to arrows F -Fig. 4, coming from a compressor, not shown. The conduit 17 has a part 18 that ascends in the chamber 14 above the level of the medicament 15 and which terminates in a conical shape (or also cylindrical) with an orifice summit 19. On the ascending part 18 of the air conduit 17 a cap 20 is fitted that delimits with said part 18 an annular cavity 21, which is open at the bottom towards the chamber 14 and dips in the medicament 15 and, at the top, flows into an injector nozzle 22 placed in line and on a level with the orifice 19 of the conduit 17. From the cap 21 two arms 23 extends upwards supporting a so-called "pisper" consisting in a beam 24 and a break-jet cylinder 25 facing towards the injector nozzle 22 and eventually an appendix 26 facing upwards.

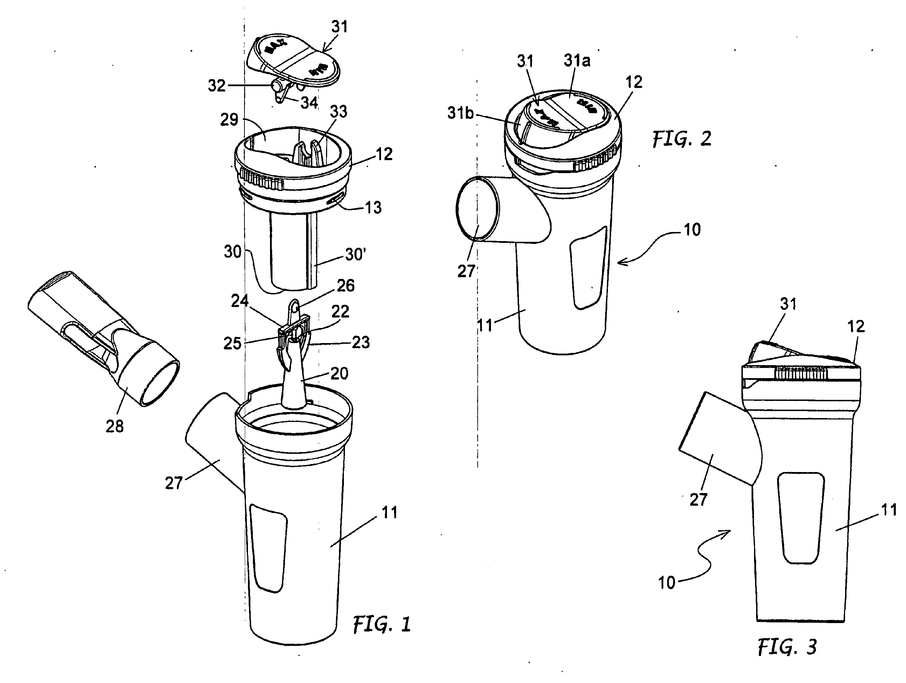

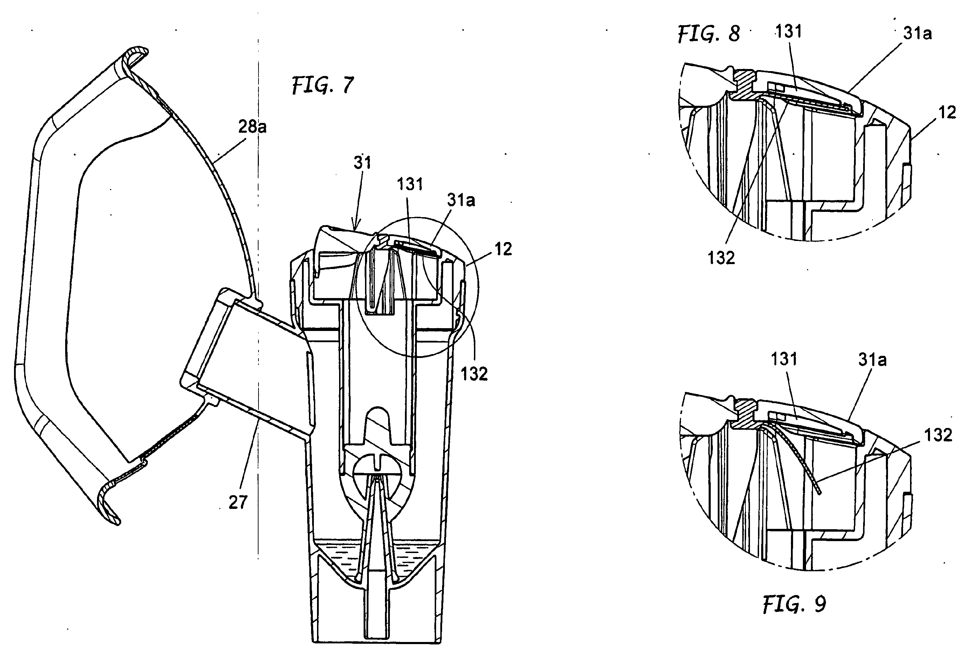

In the example represented, the lower element 11 of the ampoule body 10 has an exit conduit 27 of the medicament from the chamber 14 towards the utilization, a mouthpiece Fig. 1 or a inhalation mask 28a -Fig. 7- or a nose adaptor being connectable to the conduit 28, to facilitate the inhalation of the medicament on the part of a patient.

The cover 12 associated with the lower element 11 of the ampoule body 10 has an upper hollow 29 with a bottom on a level with which there is the entry of a feed conduit 30 that extends downwards in the chamber 14 as far as the proximity of the "pisper". The conduit 30 has two radial grooves 30' by means of which it unambiguously connects with the two arms 23 of the cap 20 defining the injector nozzle 22, so as to always guarantee the same direction of the same "pisper".

This assembly, furthermore, facilitates the normal usage and washing operations of the ampoule carried out by the user, limiting the number of small items to be separately manipulated and furthermore easy to lose. In fact, thanks to this arrangement, from the point of view of the user, the ampoule is made up of only two parts: "body, or lower part, and cover, or upper part".

The feed conduit 30 is designed to allow the entrance from outside of a flow of ambient air in the chamber 14. This flow of air can be varied by means of a speed regulator/variator. For this purpose, on a level with the hollow 29 of the cover, a key or selection push-button 31 is located, which is provided with pins 32 defining an oscillation axis and by means of which the push-button is assembled balancing on supporting forks 33, also with the possibility of separating it for washing operations of the components of the ampoule.

The selection push-button 31 has essentially a first portion 31 a and a second portion 31b on opposite parts of its oscillation axis and is moveable at least from a first position -Fig. 4- to a second position -Fig. 5- delimitated by at least a stop tang 34 integral with the push-button itself and resting, in the example shown, alternatively in two or more stop and positioning seats 35 e 36.

In the first position, the selection push-button 31 obstructs by its first portion 31a the entry of the delivery conduit 30, reducing the contribution of ambient air to the chamber 14 for a first, minor, nebulization speed and delivery of medicament towards the patient through the exit conduit with mouthpiece, inhalation mask or nasal adapter. In this case, in fact, the air enters from outside and flows to the feed conduit 30, then to the chamber 14, only through the gaps around the push button, between the latter and the lateral surfaces of the hollow 29, furthermore reducing the noise of the system.

In the second position, the first portion 31 a of the selection push-button 31 allows on the other hand a free access of the ambient air to the feed conduit 30 for an increase in the flow of air to the chamber 14, creating in this way the condition for a second, greater, atomization and delivery speed of the medicament towards the patient.

Furthermore, between the extreme positioning seats 35, 36 other intermediate positioning seats can be provided so as to establish as many different opening positions of the push-button 31 and by this further various delivery speeds of the medicament.

The portion 31a of the key or selection push-button 31 designed to obstruct or not allow the entry of the feed conduit 30, presents one or more passages 131 in line with which an additional valve 132 is associated with the push-button -Fig. 7-, for example the membrane type, that is flexible, moveable between a closed position -Fig. 8- and an open position - Fig. 9.

When the selection push button 31 is in the first position for a first, minor, nebulization speed and as long as the additional valve 132 is in the closed position as in Fig. 8, said valve has very little influence over the useful feed of medicament at that speed. However with the inhalation of the medicament on the part of the patient, the additional valve 132 tends to open as in Fig. 9, allowing in this way the air to enter from outside through the passages 131 and by this the formation of an aerosol richer in medicament even if the selection push-button remains in the first position corresponding to the lower running speed of the device.

When on the other hand the selection push-button 31 is in the second higher nebulization and medicament feed speed position the additional valve remains completely inactive.

As said above, to the exit conduit 27 of the medicament from the chamber 14 of the ampoule either a mouthpiece 28 or a inhalation mask 28a or a nasal adapter is applicable. According to the invention at least one of these parts, in particular the inhalation mask 28a, can be made of a bi-material and provided with at least a dispersion limiter means 133 with the function of limiting the dispersion, that is to say a loss of medicament towards the external of the half mask.

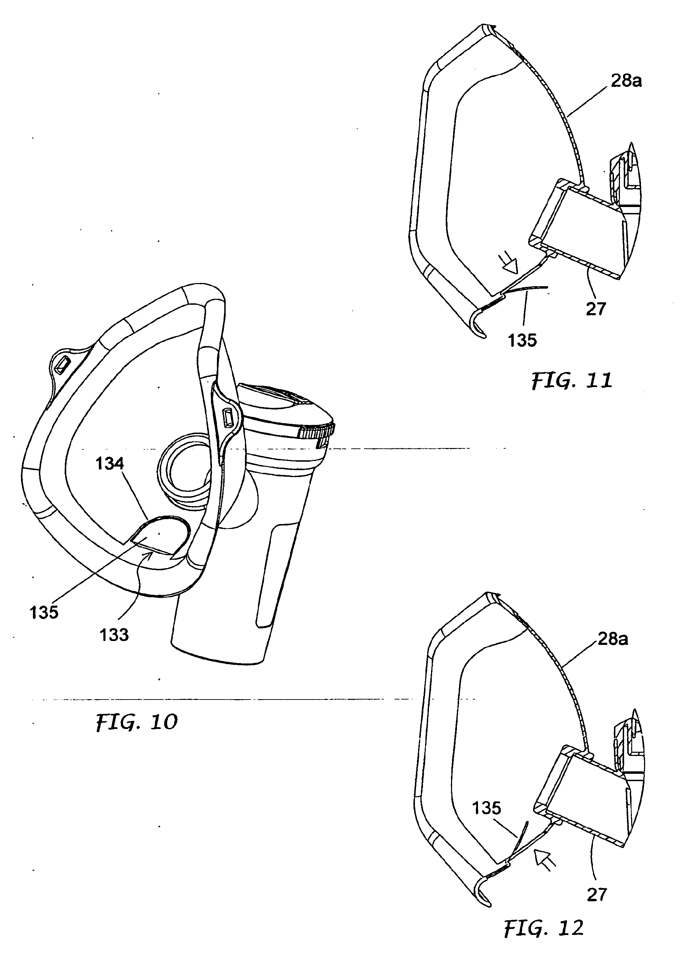

In the example shown, said dispersion limiter means 133 -Fig. 9- is in the form of an opening variable in width defined by at least a split 134 provided in at least one thin zone of the wall of the inhalation mask 28a so as to delimit at least a flexible portion 135, which starting from a coplanar arrangement to the surrounding wall of the inhalation mask it can bend both towards the inside and towards the outside of the mask itself. Should there be a lack of stress, the flexible elastic portion 135 remains in that coplanar position, which corresponds to the width of the opening 133 which is null or nevertheless minimal. In the course of a treatment, during the inhalation and expiration phases of the patient, flexions are produced in the elastic flexible portion 135 towards the inside and outside of the inhalation mask, respectively, with a corresponding variation in the width of the opening as shown in Figs. 11 and 12, respectively. In the intermediate phases between inhalation and expiration the elastic-flexible sector 135 places itself in a coplanar position to the surrounding wall of the inhalation mask, limiting in this way the dispersion of medicament.

To be noted that when the selection push-button 31 of the ampoule is provided with an additional valve 132 there must be a correlated rigidity between that valve and dispersion limiter means 133 in the sense that work wise the valve 132 on the selection push-button has to open before the dispersion limiter means 133.

## Claims

1. Ampoule nebuliser for aerosol therapy devices, comprising
an ampoule body (10) that defines a chamber (14) designed to contain a medicament liquid to be nebulised,
a conduit (17) extending upwards from the bottom of said chamber, designed for the input of a flow of compressed air (F), terminating with a top orifice (19) and bearing a nebuliser nozzle (22) preferably overlooked by a break-jet cylinder (25),
a through conduit (30) open both towards the external and towards the nebuliser nozzle (22) for an additional flow of ambient air, and
a delivery conduit (27) that extends from said chamber (14) to deliver the nebulised medicament towards a mouthpiece (28) or a inhalation mask (28a) or a nasal adapter for use on the part of a patient
wherein on a level with the entry of said ambient air supply conduit (30) a selection push-button (31) is provided, the selection push-button (31) is movable between a first operating position obstructing at least partially the entry of said conduit (30) for the choice of a first minimal atomization speed and at least a second operating position opening the entry of said conduit (30) for the choice of a second higher atomization speed of the medicament, said positions being defined by blocking means (35, 36), where in said ampoule body (10) is made up of a lower element (11) and a top cover (12) connected to each other by screwing or by bayonet, wherein said cover (12) has an upper hollow (29) and the supply conduit (30) of ambient air extends from the bottom of said hollow downwards as far as the proximity of the nebulizer nozzle (22) and **characterized in that** said selection push-button (31) is mounted and removable in said hollow (29) above the entry of said delivery conduit and accessible on a level with said cover (12).

2. Ampoule nebuliser according to claim 1, **characterized in that** the selection push-button (31) has a portion (31 a) designed to obstruct at least partially the entry of said feed conduit (30) provided with one or more passages (131) for the entrance of ambient air, said selection push-button being associated with an additional valve (132) movable between a closed and an open position of said passages (131) when the selection push-button is in said first operating position and in answer to every inhalation phase of the patient.

3. Ampoule nebuliser according to claim 1 or 2, **characterized in that** said delivery conduit (27) is associated with a inhalation mask (28a) provided with a limiter means (133) to reduce the dispersion of the medicament towards the outside between the inhalation and expiration phases of the patient.

4. Ampoule nebuliser according to claim 2, wherein said additional valve (132) is made up of at least an elastic-flexible membrane.

5. Ampoule nebuliser according to claim 3, wherein said limiter dispersion means (133) is made up of a variable width opening defined by a split (134) provided in a thin zone of the wall of the inhalation mask (28a) so as to delimit at least a flexible portion (135) which is coplanar to said zone of the wall of the inhalation mask along a minimum width of the opening and is flexible both towards the inside and towards the outside of the inhalation mask so as to vary the width of the opening in answer to the inhalation and expiration phases of the patient.

6. Ampoule nebuliser according to any one of the previous claims, wherein between said first and said second opening position of the selection push button (31) one or more intermediate opening positions are established by respective stopping means so as to create further atomisation speed of the medicament.

7. Ampoule nebuliser according to claim 1, wherein said selection push-button (31) has a stop tang (34) selectively engaging with said blocking means (35, 36) to define the various opening positions and corresponding different atomisation speeds of the medicament.

8. Ampoule nebuliser according to any one of the previous claims, wherein, in said first position, the selection push-button(31) at least obstructs partially the entry of the supply conduit (30), reducing the contribution of ambient air to the chamber (14) for a first, low, output atomisation speed of the medicament, and reducing the noise of the ampoule device, consequently the air enters from outside and flows in the supply conduit (30) to the chamber (14), only through the interstices between the push button and the lateral surface of the hollow, and wherein in said second position the selection push button (31) allows free access of the ambient air to the feed conduit (30), for a higher flow of air to the chamber and a higher atomisation speed of the medicament.

9. Ampoule nebuliser according to any one of the previous claims, wherein the entry of the ambient air supply conduct has two radial grooves (30') designed to receive two opposite parts of a means defining the nebulizer nozzle for a precise and univocal positioning of the means in said chamber.

## Patentansprüche

1. Zcrstäubcrampulle für Aerosoltherapiegeräte, umfassend
- einen Ampullenkörper (10), welcher eine Kammer (14) definiert, die zur Aufnahme des zu zerstäubenden Flüssigmedikamentes vorgesehen ist,
- eine Leitung (17), welche sich nach oben vom Boden der besagten Kammer erstreckt, zum Eintreten einer Druckluftströmung (F) vorgesehen ist, mit einer oberen Öffnung (19) endet und eine vorzugsweise von einem Strahlbrecher-Zylinder (25) überragte Atomisierdüse aufweist,
- eine Durchleitung (30), die jeweils nach außen und zu der Atomisierdüse (22) hin geöffnet ist und für eine zusätzliche Strömung von Umgebungsluft vorgesehen ist, und
- eine Zuführleitung (27), welche sich von der besagten Kammer (14) zum Zuführen des zerstäubten Medikamentes an ein Mündstück (28) oder eine Inhalationsmaske (28a) oder einen Nasenadapter erstreckt, der zum Auftragen auf einer Körperpartie des Patienten verwendet wird,
- wobei auf Höhe des Einlasses der besagten Umgebungsluft-Versorgungsleitung (30) eine Wähldrucktaste (31) vorgesehen ist, wobei die Wähldrucktaste (31) zwischen einer ersten Betriebsposition, in der sie den Einlass der besagten Leitung (30) mindestens teilweise zum Auswählen einer ersten minimalen Zerstäubergeschwindigkeit obstruiert, und zumindest einer zweiten Betriebsposition, in der sie den Einlass der besagten Leitung (30) zum Auswählen einer zweiten höheren Zerstäubergeschwindigkeit des Medikamentes öffnet, bewegbar ist, wobei die besagten Positionen durch Sperrmittel (35, 36) definiert werden, wobei der besagte Ampullenkörper (10) von einem Unterelement (11) und einer oberen Abdeckung (12) gebildet ist, die durch Schrauben- oder Bajonettverschluss miteinander verbunden sind, wobei die besagte Abdeckung (12) eine obere Vertiefung (29) aufweist und die Versorgungsleitung (30) für die Umgebungsluft sich aus dem Boden der besagten Vertiefung bis in der Nähe der Atomisierdüse (22) erstreckt, **dadurch gekennzeichnet, dass** die besagte Wähldrucktaste (31) in der besagten Vertiefung (29) oberhalb des Einlasses der besagten Zuführleitung lösbar eingebaut sowie auf Höhe der besagten Abdeckung (12) zugänglich ist.

2. Zerstäuberampulle nach Anspruch 1, **dadurch gekennzeichnet, dass** die Wähldrucktaste (31) einen Abschnitt (31a) aufweist, welcher zum zumindest teilweisen Verschließen des Einlasses der besagten Versorgungsleitung (30) vorgesehen und mit einem oder mehreren Durchgängen (131) zum Einlassen der Umgebungsluft versehen ist, wobei die besagte Wähldrucktaste einem zusätzlichen Ventil (132) zugeordnet ist, das zwischen einer geschlossenen Position und einer geöffneten Position der besagten Durchgänge (131) bei in der besagten ersten Betriebsposition sich befindender Wähldrucktaste und in Antwort auf jede Inhalationsphase des Patienten bewegbar ist.

3. Zerstäuberampulle nach Anspruch 1 oder 2, **dadurch gekennzeichnet, dass** die besagte Zuführleitung (27) einer Inhalationsmaske (28a) zugeordnet ist, die mit einem Begrenzungsmittel (133) versehen ist, um eine Dispersion des Medikamentes zwischen einer Inhalationsphase und einer Exspirationsphase nach außen zu reduzieren.

4. Zerstäuberampulle nach Anspruch 2, wobei das besagte zusätzliche Ventil (132) aus mindestens einer biegeelastischen Membran gebildet wird.

5. Zerstäuberampulle nach Anspruch 3, wobei das besagte Dispersions-Begrenzungsmittel (133) aus einer Öffnung mit variabler Weite gebildet ist, welche durch eine Spalte (134) definiert wird, die in einer dünnen Wandzone der Inhalationsmaske (28a) vorgesehen ist, um mindestens einen flexiblen Abschnitt (135) zu begrenzen, welcher komplanar zu der besagten Wandzone der Inhalationsmaske entlang einer Mindestweite der Öffnung angeordnet ist und nach dem Inneren und nach dem Äußeren der Inhalationsmaske hin flexibel ist, um die Weite der Öffnung in Antwort auf die Inhalationsphase und Exspirationsphase des Patienten zu variieren.

6. Zerstäuberampulle nach einem der vorhergehenden Ansprüche, wobei zwischen der besagten ersten und der besagten zweiten geöffneten Position der Wähldrucktaste (31) eine oder mehrere Zwischen-Öffnungspositionen durch entsprechende Haltemittel gebildet werden, um eine höhere Zerstäubungsgeschwindigkeit des Medikamentes zu schaffen.

7. Zerstäuberampulle nach Anspruch 1, wobei die besagte Wähldrucktaste (31) eine Rastzunge (34) aufweist, die in das besagte Sperrmittel (35, 36) wahlweise eingreift, um die verschiedenen Öffnungspositionen und entsprechend die verschiedenen Zerstäubungsgeschwindigkeiten des Medikamentes einzustellen.

8. Zerstäuberampulle nach einem der vorhergehenden Ansprüche, wobei die Wähldrucktaste (31) in der besagten ersten Position den Einlass der Versorgungsleitung (30) mindestens teilweise obstruiert und dadurch den Beitrag an Umgebungsluft in die Kammer (14) für eine erste langsame Abgabezerstäubungsgeschwindigkeit reduziert sowie die Gerauschbelastung der Ampulieneihrichtung mindert und infolgedessen die Luft von außen einströmt und in der Versorgungsleitung (30) bis zu der Kammer (14) fließt, nur durch Zwischenräume zwischen der Drucktaste und der Seitenfläche der Vertiefung strömend, und wobei in der besagten zweiten Position die Wähldrucktaste (31) den freien Zugang der Umgebungsluft zu der Versorgungsleitung (30) zulässt, um eine höhere Luftströmung in die Kammer und eine höhere Zerstäubungsgeschwindigkeit des Medikamentes zu bewirken.

9. Zerstäuberampulle nach einem der vorhergehenden Ansprüche, wobei der Einlass der Umgebungsluft-Versorgungsleitung zwei Radialnuten (30') aufweist, welche zum Aufnehmen zweier gegenüberliegender Teile eines Mittels vorgesehen sind, welche Teile die Atomisierdüse definieren, um eine exakte und eindeutige Positionierung des Mittels in der besagten Kammer zu bewirken.

## Revendications

1. Cuve de nébulisation pour dispositifs d'aérosolthérapie, comprenant
un corps (10) de cuve qui définit une chambre (14) destinée à contenir un médicament liquide à nébuliser,
un conduit (17) s'étendant vers le haut à partir du fond de ladite chambre, conçu pour l'entrée d'un flux d'air comprimé (F), se terminant par un orifice supérieur (19) et portant une buse de nébulisation (22), de préférence surmontée par un cylindre brise-jet (25),
un conduit traversant (30) ouvert à la fois vers l'extérieur et vers la buse de nébulisation (22) pour un flux supplémentaire d'air ambiant, et
un conduit de distribution (27) qui s'étend à partir de ladite chambre (14) pour distribuer le médicament pulvérisé vers une un embout buccal (28) ou un masque d'inhalation (28a) ou un adaptateur nasal pour être utilisé par un patient
dans lequel au niveau de l'entrée dudit conduit (30) d'alimentation de l'air ambiant, est prévu un poussoir de sélection (31), le poussoir de sélection (31) est mobile entre une première position de fonctionnement pour obstruer au moins partiellement l'entrée dudit conduit (30) pour le choix d'une première vitesse de nébulisation minimal et au moins une deuxième position de fonctionnement pour ouvrir l'entrée dudit conduit (3) pour le choix d'une seconde vitesse de nébulisation supérieure du médicament, lesdites positions étant définies par des moyens de blocage (35, 36), dans lequel ledit corps (10) de cuve est composé d'un élément inférieur (11) et un couvercle supérieur (12) reliés entre eux par vissage ou par une monture à baïonnette, dans lequel ledit couvercle (12) a un creux supérieur (29) et le conduit (30) d'alimentation de l'air ambiant s'étend à partir de la partie inférieure dudit creux vers le bas jusqu'à proximité de la buse de nébulisation (22) et **caractérisée en ce que** ledit poussoir de sélection (31) est monté, et amovible, dans ledit creux (29) au-dessus de l'entrée dudit conduit de distribution et accessible au niveau dudit couvercle (12).

2. Cuve de nébulisation selon la revendication 1, **caractérisée en ce que** le poussoir de sélection (31) comporte une partie (31a) destinée à obstruer au moins partiellement l'entrée dudit conduit d'alimentation (30) muni d'un ou plusieurs passages (131) pour l'entrée de l'air ambiant, ledit poussoir de sélection étant associé à une soupape supplémentaire (132) pouvant se déplacer entre une position fermée et une ouverte desdits passages (131) lorsque le poussoir de sélection se trouve dans ladite première position de fonctionnement et en réponse à chaque phase d'inhalation du patient.

3. Cuve de nébulisation selon la revendication 1 ou 2, **caractérisée en ce que** ledit conduit de distribution (27) est associé à un masque d'inhalation (28a) muni d'un moyen de limitation (133) afin de réduire la dispersion du médicament vers l'extérieur entre les phases de inhalation et de expiration du patient.

4. Cuve de nébulisation selon la revendication 2, dans laquelle ladite soupape supplémentaire (132) est composée d'au moins une membrane élastique flexible.

5. Cuve de nébulisation selon la revendication 2, dans laquelle ledit moyen de limitation (133) de la dispersion est composé d'une ouverture de largeur variable définie par une fente (134) prévue dans une zone mince de la paroi du masque d'inhalation (28a) de manière à délimiter au moins une portion flexible (135) qui est normalement coplanaire à ladite zone de la paroi du masque d'inhalation le long d'une largeur minimum de l'ouverture et est flexible à la fois vers l'intérieur et vers l'extérieur du masque d'inhalation de manière à faire varier la largeur de l'ouverture en réponse aux phases de inhalation et de expiration du patient.

6. Cuve de nébulisation selon l'une quelconque des revendications précédentes, dans laquelle entre ladite première et ladite seconde position d'ouverture du poussoir de sélection (31) une ou plusieurs positions d'ouverture intermédiaires sont établies par des respectifs moyens d'arrêt de manière à augmenter davantage la vitesse d'atomisation du médicament.

7. Cuve de nébulisation selon la revendication 1, dans laquelle ledit poussoir de sélection (31) a une patte d'arrêt (34) venant en prise sélectivement avec lesdits moyens (35, 36) de blocage pour définir les différentes positions d'ouverture et différentes vitesses d'atomisation du médicament correspondantes.

8. Cuve de nébulisation selon l'une quelconque des revendications précédentes, dans laquelle, dans ladite première position, le poussoir de sélection (31) obstrue au moins partiellement l'entrée du conduit d'alimentation (30) en réduisant l'apport d'air ambiant à la chambre (14) pour une première faible vitesse d'atomisation de sortie du médicament et en réduisant le bruit du dispositif de cuve, par conséquent l'air pénètre de l'extérieur et coule dans le conduit d'alimentation (30) vers la chambre (14) seulement à travers les interstices entre le poussoir et la surface latérale du creux, et dans laquelle, dans ladite seconde position, le poussoir de sélection (31) permet le libre accès de l'air ambiant vers le conduit d'alimentation (30), pour un débit plus élevé d'air à la chambre et une vitesse d'atomisation plus élevée du médicament.

9. Cuve de nébulisation selon l'une quelconque des revendications précédentes, dans laquelle l'entrée du conduit d'alimentation de l'air ambiant a deux rainures radiales (30') conçues pour recevoir deux parties opposées d'un moyen définissant la buse de nébulisation pour un positionnement précis et univoque des moyens dans ladite chambre.
